(19) Europäisches Patentamt
European Patent Office
Office européen des brevets

(11) EP 1 795 194 A2

(12) **DEMANDE DE BREVET EUROPEEN**

(43) Date de publication:
**13.06.2007 Bulletin 2007/24**

(51) Int Cl.:
***A61K 31/44*** *(2006.01)*

(21) Numéro de dépôt: **07105573.5**

(22) Date de dépôt: **28.01.1998**

(84) Etats contractants désignés:
**AT BE CH DE DK ES FI FR GB GR IE IT LI LU MC NL PT SE**

(30) Priorité: **28.01.1997 FR 9700870**

(62) Numéro(s) de document de la (des) demande(s) initiale(s) en application de l'article 76 CBE:
**98904238.7 / 0 969 835**

(71) Demandeur: **Sanofi-Aventis**
**75013 Paris (FR)**

(72) Inventeurs:
- **Maruani, Jeanne**
**34570, VAILHAUQUES (FR)**
- **Soubrie, Philippe**
**deceased (FR)**

(74) Mandataire: **Gillard, Marie-Louise et al**
**Cabinet Beau de Loménie**
**158, rue de l'Université**
**75340 Paris Cédex 07 (FR)**

Remarques:
Cette demande a été déposée le 03 - 04 - 2007 comme demande divisionnaire de la demande mentionnée sous le code INID 62.

# (54) Utilisation d'un antagoniste des récepteurs CB1 associé à un bêta3-agoniste pour la préparation de médicaments utiles pour traiter l'appétence

(57) L'invention concerne l'utilisation d'un antagoniste des récepteurs CB1 en association avec un agoniste du récepteur $\beta_3$-adrénergique, pour la préparation de médicaments utiles dans le traitement des troubles de l'appétence.

EP 1 795 194 A2

## Description

La présente invention concerne une nouvelle utilisation des antagonistes des récepteurs aux cannabinoïdes centraux dits récepteurs CB1.

Plus particulièrement, l'invention se rapporte à l'utilisation des antagonistes des récepteurs CB1 pour la préparation de médicaments utiles pour traiter les troubles de l'appétence. Les médicaments utiles pour traiter les troubles de l'appétence sont destinés à réguler les désirs de consommation, en particulier pour la consommation de sucres, de carbohydrates, d'alcool ou de drogues et plus généralement d'ingrédients appétissants.

Dans la présente description et dans les revendications, on désigne par troubles de l'appétence,

- les troubles liés à une substance, et notamment abus d'une substance et/ou dépendance à une substance,
- les troubles des conduites alimentaires, notamment susceptibles d'entraîner un surpoids, quel qu'en soit l'origine, par exemple : boulimie, appétence aux sucres, diabète non insulino-dépendant.

Par substances on entend des ingrédients appétissants tels que les sucres, carbohydrates, alcools ou drogues.

La présente invention se rapporte donc également à l'utilisation d'un antagoniste des récepteurs CB1 pour préparer des médicaments utiles dans le traitement de la boulimie et de l'obésité y compris l'obésité liée au diabète de type II (diabète non insulino-dépendant) ou plus généralement de toute maladie se traduisant par un surpoids du patient, ainsi que dans le traitement de l'abus de drogues ou de la dépendance aux drogues.

Le delta-9 tétrahydrocannabinol ou $\Delta^9$-THC est le principal constituant actif extrait de Cannabis sativa (Tuner, 1985 ; In Marijuana 84, Ed. Harvey, DY, IRL Press, Oxford).

Les effets des cannabinoïdes sont dûs à une intéraction avec des récepteurs spécifiques de haute affinité couplés aux protéines G. Deux types de récepteurs sont actuellement décrits : les récepteurs CB1, présents majoritairement au niveau du système nerveux central (Devane et al., Molecular Pharmacology, 1988, 34, 605-613) et les récepteurs CB2 présents dans le système immunitaire (Nye et al., The Journal of Pharmacology and Experimental Therapeutics, 1985, 234, 784-791 ; Kaminski et al., 1992, Molecular Pharmacology, 42, 736-742 ; Munro et al., Nature, 1993, 365, 61-65). La caractérisation de ces récepteurs a été rendue possible par la mise au point de ligands synthétiques tels que le CP 55,940 (J. Pharmacol. Exp. Ther., 1988, 247, 1046-1051), le WIN 55212-2 (J. Pharmacol. Exp. Ther., 1993, 264, 1352-1363) et, plus récemment, par la découverte d'un antagoniste sélectif des récepteurs CB1 : le SR 141716 A (M. Rinaldi-Carmona et al., FEBS Lett., 1994, 350, 240-244).

Des familles de composés ayant une affinité pour les récepteurs aux cannabinoïdes ont été décrites dans plusieurs brevets ou demandes de brevets, notamment la demande européenne EP-576 357, qui décrit des dérivés du pyrazole, et la demande WO 96/02248 qui décrit notamment des dérivés du benzofurane.

Plus particulièrement, le N-pipéridino-5-(4-chlorophényl)-1-(2,4-dichlorophényl)-4-méthylpyrazole-3-carboxamide, également dénommé SR 141716, de formule :

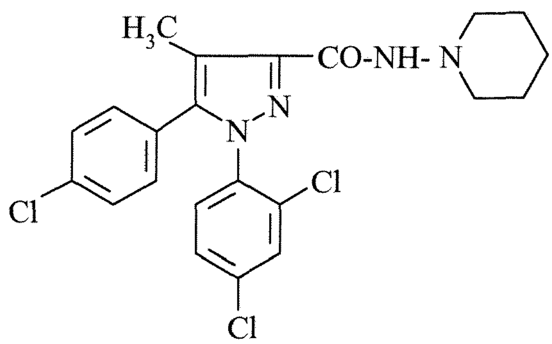

(I)

ses sels pharmaceutiquement acceptables et leurs solvates, sont décrits dans la demande de brevet européen EP-656 354, comme antagonistes des récepteurs centraux $CB_1$.

Le SR 141716 A est le chlorhydrate du SR 141716.

Il est connu que le delta-9 tétrahydrocannabinol dont la dénomination commune internationale est Dronabinol est utilisé pour le traitement de l'anorexie, notamment chez les patients souffrant du SIDA (J. Pain Symptom Manage, 1995, 10 (2), 89-97) ou du cancer (J. Palliat. Care., 1994, 10 (1), 14-18).

De plus il est décrit que le SR 141716 et ses sels qui sont des antagonistes des récepteurs centraux aux cannabinoïdes peuvent être utilisés pour le traitement des troubles de l'appétit, notamment en tant qu'anorexigène, et dans le traitement des troubles liés à l'utilisation de substances psychotropes.

Les anorexigènes classiques provoquent une diminution de l'appétit qui est généralement indépendante des substances alimentaires à consommer.

De façon surprenante, on a maintenant trouvé que les antagonistes des récepteurs CB1 ont une propriété spécifique en agissant de façon élective sur les troubles des comportements consommatoires vis-à-vis des substances appétissantes.

Ainsi, l'administration d'un antagoniste des récepteurs CB1 permet de réguler le désir de consommation pour des éléments non essentiels de l'alimentation tels que les sucres en excès, les carbohydrates en excès, l'alcool ou les drogues.

En effet, après avoir conduit des essais chez l'animal, on a observé un comportement nouveau de celui-ci : l'animal ne manifeste plus d'appétence spontanée pour l'ingrédient qui lui procure habituellement un plaisir tel que le sucre ou l'alcool, par exemple. Ce manque d'appétence se manifeste également lorsque l'animal a été prétraité par un neuropeptide connu pour augmenter l'appétit tel que le neuropeptide Y (NPY) par exemple.

Selon un de ses aspects, la présente invention concerne l'utilisation d'un antagoniste des récepteurs CB1 pour la préparation de médicaments utiles pour le traitement des troubles de l'appétence.

Les antagonistes des récepteurs CB1 appropriés aux fins de l'invention sont en particulier les composés de formule :

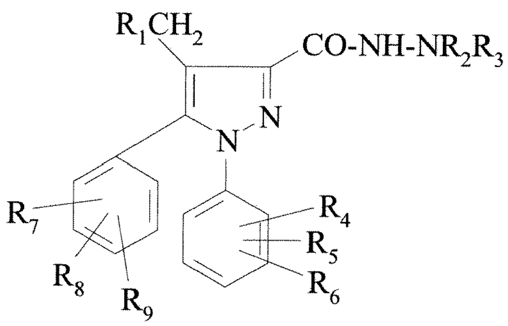

(II)

dans laquelle :

- $R_1$ représente l'hydrogène, un fluor, un hydroxy, un $(C_1\text{-}C_5)$alcoxy, un $(C_1\text{-}C_5)$alkylthio, un hydroxy$(C_1\text{-}C_5)$alcoxy, un groupe $-NR_{10}R_{11}$, un cyano, un $(C_1\text{-}C_5)$alkylsulfonyle ou un $(C_1\text{-}C_5)$alkylsulfinyle ;
- $R_2$ et $R_3$ représentent un $(C_1\text{-}C_4)$alkyle ou ensemble avec l'atome d'azote auquel ils sont liés constituent un radical hétérocyclique, saturé ou insaturé, de 5 à 10 chaînons, non substitué ou substitué une ou plusieurs fois par un $(C_1\text{-}C_3)$alkyle ou par un $(C_1\text{-}C_3)$alcoxy ;
- $R_4$, $R_5$, $R_6$, $R_7$, $R_8$, $R_9$ représentent chacun indépendamment l'hydrogène, un halogène ou un trifluorométhyle, et lorsque $R_1$ représente un fluor, $R_4$, $R_5$, $R_6$, $R_7$, $R_8$ et/ou $R_9$ peuvent également représenter un fluorométhyle ; à la condition que l'un au moins des substituants $R_4$ ou $R_7$ soit différent de l'hydrogène ;
- $R_{10}$ et $R_{11}$ représentent chacun indépendamment l'hydrogène ou un $(C_1\text{-}C_5)$alkyle ou $R_{10}$ et $R_{11}$ ensemble avec l'atome d'azote auquel ils sont liés constituent un radical hétérocyclique choisi parmi pyrrolidin-1-yle, pipéridin-1-yle, morpholin-4-yle et pipérazin-1-yle, non substitué ou substitué par un $(C_1\text{-}C_4)$alkyle ;

ainsi que leurs sels et leurs solvates.

Plus particulièrement, la présente invention concerne l'utilisation du N-pipéridino-5-(4-chlorophênyl)-1-(2,4-dichlorophényl)-4-méthylpyrazole-3-carboxamide, de ses sels pharmaceutiquement acceptables et de leurs solvates pour la préparation de médicaments utiles pour le traitement des troubles de l'appétence.

Selon la présente invention, on peut également utiliser les antagonistes des récepteurs CB1 en association avec un autre principe actif pour préparer des médicaments utiles pour le traitement des troubles de l'appétence, notamment pour le traitement des troubles des conduites alimentaires ; on peut utiliser une composition pharmaceutique comprenant en association un antagoniste des récepteurs CB1 et un composé régulateur des désordres métaboliques, notamment un agoniste du récepteur $\beta_3$-adrénergique ci-après dénommé $\beta_3$-agoniste.

Ainsi la présente invention a également pour objet des compositions pharmaceutiques contenant un antagoniste des récepteurs CB1 et un régulateur des désordres métaboliques, comme par exemple des hypolipémiants, hypolydémiants ou lipolytiques. Plus particulièrement, la présente invention a pour objet des compositions pharmaceutiques contenant un antagoniste des récepteurs CB1 et un $\beta_3$-agoniste.

Des $\beta_3$-agonistes utiles pour l'utilisation selon la présente invention sont les composés de formule :

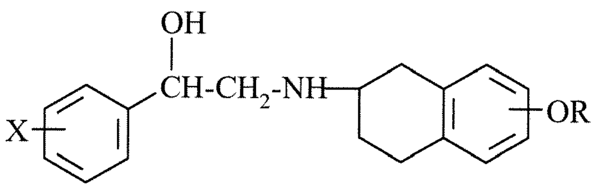

(III)

dans laquelle

- X représente l'hydrogène, un halogène, un trifluorométhyle ou un $(C_1-C_4)$alkyle;
- R représente l'hydrogène ou un méthyle, non substitué ou substitué par un carboxy ou un alcoxycarbonyle dans lequel l'alcoxy est en $(C_1-C_6)$,

et leurs sels pharmaceutiquement acceptables, indiqués dans EP 0 211 721 et EP 0 303 546 comme spasmolytiques intestinaux.

Parmi les composés de formule (III), les composés:

* 2-[(7-hydroxy-1,2,3,4-tétrahydronapht-2-yl)amino]-1-phényléthanol;
* 2-[(7-hydroxy-1,2,3,4-tétrahydronapht-2-yl)amino]-1-(3-chlorophényl)éthanol;
* 2-[(7-éthoxycarbonylméthoxy-1,2,3,4-tétrahydronapht-2-yl)amino]-1-(3-chlorophényl) éthanol;
* 2-[(7-éthoxycarbonylméthoxy-1,2,3,4-tétrahydronapht-2-yl)amino]-1-phényléthanol;
* (1R,2'RS)-2-[(7-hydroxy-1,2,3,4-tétrahydronapht-2-yl)amino]-1-phényléthanol;
* (1S,2'RS)-2-[(7-hydroxy-1,2,3,4-tétrahydronapht-2-yl)amino]-1-phényléthanol;
* (+)-(1R)-2-[(7-hydroxy-1,2,3,4-tétrahydronapht-2-yl)amino]-1-phényléthanol;
* (+)-(1S)-2-[(7-hydroxy-1,2,3,4-tétrahydronapht-2-yl)amino]-1-phényléthanol;
* (-)-(1R)-2-[(7-hydroxy-1,2,3,4-tétrahydronapht-2-yl)amino]-1-phényléthanol;
* (-)-(1S)-2-[(7-hydroxy-1,2,3,4-tétrahydronapht-2-yl)amino]-1-phényléthanol;
* N-[(2S)-7-éthoxycarbonylméthoxy-1,2,3,4-tétrahydronapht-2-yl]-(2R)-2-(3-ehlorophényl)-2-hydroxyéthanamine;
* N-[(2R)-7-méthoxycarbonylméthoxy-1,2,3,4-tétrahydronapht-2-yl]-(2R)-2-(3-chlorophényl}-2-hydroxyéthanamine;

et leurs sels pharmaceutiquement acceptables sont des composés particulièrement avantageux.

La N-[(2S)-7-éthoxycarbonylméthoxy-1,2,3,4-tétrahydronapht-2-yl]-(2R)-2-(3-chlorophényl)-2-hydroxyéthanamine (SR 58611) et ses sels pharmaceutiquement acceptables sont tout particulièrement avantageux, notamment son sel avec l'acide chlorhydrique, le SR 58611 A.

D'autres $\beta_3$-agonistes utiles pour l'utilisation selon la présente invention sont les composés de formule :

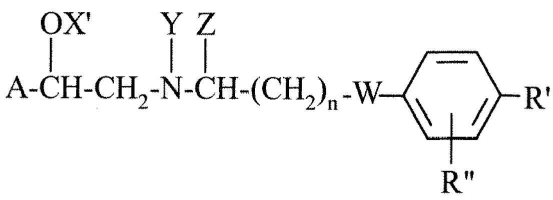

(IV)

dans laquelle

- n est 1, 2 ou 3;
- A représente un benzofuran-2-yle ou un phényle non substitué ou substitué par un ou deux atomes d'halogène ou par un $(C_1-C_4)$alkyle ou un trifluorométhyle;
- R' représente
  - l'hydrogène;
  - un $(C_1-C_6)$alkyle;
  - un groupe fonctionnel choisi parmi un groupe hydroxy, $(C_1-C_6)$alcoxy, $(C_2-C_6)$alcényloxy, $(C_2-C_6)$alcynyloxy, $(C_3-C_8)$cycloalkyloxy, $(C_3-C_8)$cycloalkyl$(C_1-C_6)$alcoxy, benzyloxy, phénoxy, mercapto, $(C_1-C_6)$alkylthio, $(C_2-C_6)$

alcénylthio, $(C_2-C_6)$alcynylthio, $(C_3-C_8)$cycloalkylthio, $(C_3-C_8)$cycloalkyl$(C_1-C_6)$alkylthio, benzylthio, phénylthio, $(C_1-C_6)$alkylsulfinyle, $(C_2-C_6)$alcénylsulfinyle, $(C_2-C_6)$alcynylsulfinyle, $(C_3-C_8)$cycloalkylsulfinyle, $(C_3-C_8)$cycloalkyl$(C_1-C_6)$alkylsulfinyle, benzylsulfinyle, phénylsulfinyle, $(C_1-C_6)$alkylsulfonyle, $(C_2-C_6)$alcénylsulfonyle, $(C_2-C_6)$alcynylsulfonyle, $(C_3-C_8)$cycloalkylsulfonyle, $(C_3-C_8)$cyeloalkyl$(C_1-C_6)$alkylsulfonyle, benzylsulfonyle, phénylsulfonyle, cyano, nitro, amino non substitué ou substitué par un ou deux radicaux, identiques ou différents, choisis parmi les groupes $(C_1-C_6)$alkyle, $(C_2-C_6)$alcényle, $(C_2-C_6)$alcynyle, $(C_3-C_8)$cycloalkyle, $(C_3-C_8)$cycloalkyl $(C_1-C_6)$alkyle, benzyle et phényle, carboxy, alcoxycarbonyle dont l'alcoxy est en $(C_1-C_6)$, $(C_2-C_6)$alcényloxycarbonyle, $(C_2-C_6)$alcynyloxycarbonyle, $(C_3-C_8)$cycloalkyloxycarbonyle, $(C_3-C_8)$cycloalkyl$(C_1-C_6)$alcoxycarbonyle, benzyloxycarbonyle, phénoxycarbonyle ou carbamoyle non substitué ou substitué sur le groupe amino par un ou deux radicaux, identiques ou différents, choisis parmi les groupes $(C_1-C_6)$alkyle, $(C_2-C_6)$alcényle, $(C_2-C_6)$alcynyle, $(C_3-C_8)$cycloalkyle, $(C_3-C_8)$cycloalkyl$(C_1-C_6)$alkyle, benzyle et phényle;

- un groupe R"' choisi parmi un groupe $(C_1-C_6)$alkyle substitué par un groupe fonctionnel, $(C_2-C_6)$alcényle substitué par un groupe fonctionnel, $(C_2-C_6)$alcynyle substitué par un groupe fonctionnel, phényl$(C_1-C_6)$alkyle substitué sur le phényle par un $(C_1-C_6)$alkyle ou par un groupe fonctionnel, phényl$(C_2-C_6)$alcényle substitué sur le phényle par un $(C_1-C_6)$alkyle ou par un groupe fonctionnel, phényl$(C_2-C_6)$alcynyle substitué sur le phényle par un $(C_1-C_6)$alkyle ou par un groupe fonctionnel, benzyle substitué sur le phényle par un $(C_1-C_6)$alkyle ou par un groupe fonctionnel et phényle, non substitué ou substitué par un $(C_1-C_6)$alkyle ou par un groupe fonctionnel, le groupe fonctionnel étant tel que défini ci-dessus;
- un groupe O-R"', S-R"', SO-R"' ou $SO_2$-R"' dans lequel R"' est tel que défini ci-dessus;
- un groupe NR"'R°, où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- un groupe COOR"' ou un groupe CO-SR"' dans lequel R"' est tel que défini ci-dessus;
- un groupe CONR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- un groupe $SO_2$NR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- R" représente l'hydrogène; un halogène; un $(C_1-C_6)$alkyle; un groupe fonctionnel tel que défini ci-dessus; un groupe OR"', R"' étant tel que défini ci-dessus; un groupe COOR"', R"' étant tel que défini ci-dessus; ou un groupe CONR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;

- W représente une liaison directe ou un atome d'oxygène;
- X' représente l'hydrogène, un $(C_1-C_6)$alkyle ou un $(C_1-C_6)$alkylcarbonyle;
- Y représente l'hydrogène ou un groupe A'-CH(OH)-$CH_2$-, A' étant identique à A, mais autre que benzofuran-2-yle; ou bien
- X' et Y, pris ensemble, forment un groupe méthylène, éventuellement substitué par un alcoxycarbonyle dont l'alcoxy est en $(C_1-C_6)$; un groupe éthylène, éventuellement substitué par un groupe oxo; ou un groupe 1,3-propylène;
- Z représente l'hydrogène ou un $(C_1-C_6)$alkyle;

ainsi que leurs sels pharmaceutiquement acceptables, indiqués dans EP 0 255 415 comme spasmolytiques intestinaux.

D'autres $\beta_3$-agonistes encore utiles pour l'utilisation selon la présente invention sont les composés de formule :

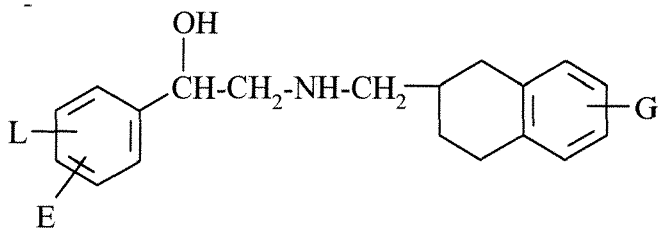

(V)

dans laquelle

- E représente l'hydrogène, un $(C_1-C_4)$alkyle, un $(C_1-C_4)$alcoxy, un phényle, un nitro, un atome d'halogène ou un

trifluorométhyle;

- L représente l'hydrogène, un ($C_1$-$C_4$)alkyle, un ($C_1$-$C_4$)alcoxy, un phényle, un nitro ou un atome d'halogène ou bien E et L , ensemble, représentent un groupe -CH=CH-CH=CH- ou -$CH_2$-$CH_2$-$CH_2$-$CH_2$- ; et
- G représente l'hydrogène, un atome de chlore, un hydroxy ou un groupe OG' où G' représente un ($C_1$-$C_4$)alkyle non substitué ou substitué par un hydroxy, ($C_1$-$C_4$)alcoxy, ($C_1$-$C_4$)alcoxycarbonyle, carboxy ou ($C_3$-$C_7$)cycloalkyle; un ($C_3$-$C_7$)cycloalkyl ou un ($C_2$-$C_4$)alcanoyle ;

ainsi que leurs sels pharmaceutiquement acceptables, indiqués dans EP 0 436 435 comme spasmolytiques intestinaux.

Parmi les composés de formule (V), la N-[(2R)-(6-hydroxy-1,2,3,4-tétrahydronapht-2-yl)méthyl]-(2R)-2-hydroxy-2-(3-chlorophényl)éthanamine (SR 59104), la N-[(2R)-(7-méthoxy-1,2,3,4-tétrahydronapht-2-yl)méthyl]-(2R)-2-hydroxy-2-(3-chlorophényl)éthanamine (SR 59119), ainsi que leurs sels pharmaceutiquement acceptables, sont des composés particulièrement avantageux.

D'autres composés $\beta_3$-agonistes avantageux selon la présente invention sont le composé BRL 35135 décrit dans EP 23385; le composé CL 316243 décrit dans US 5 061 727; le composé AZ 002 décrit dans EP 218440; le composé BMS 187257 décrit dans US 5 321 036 ; le composé ZD 7114 décrit dans EP 473 285 ; le composé RO 40-2148 décrit dans Am. J. Clin. Nutr., 1992, 55 (1, Suppl.), 249S-251S ; ainsi que les produits décrits dans les brevets/demandes de brevet suivants : WO96/35671, WO96/35670, WO96/16038, WO96/04233, WO95/33724, WO95/29159, EP659737, WO95/04047, EP516349, EP473285, EP23385, EP21636, EP 7205, JP08198866, JP08165276, JP08157470, WO96/16938, EP714883, WO96/04234, US 5 488 064, US 5 482 971, US 5 491 134, WO95/29159, WO95/33724, ZA9409874, WO95/29903, US 5 461 163, WO95/25104, EP659737, JP07112958, WO95/8527, WO95/07284, JP07025756, WO95/03289, WO95/04047, WO95/01170, WO94/29290, US 5 373 020, JP06293664, WO94/12166 et US 5 451 677.

Pour son utilisation en tant que médicament, un composé antagoniste des récepteurs CB1, seul ou en association avec un $\beta_3$-agoniste, doit être formulé en composition pharmaceutique.

Dans les compositions pharmaceutiques de la présente invention pour l'administration orale, sublinguale, sous-cutanée, intramusculaire, intraveineuse, transdermique, locale ou rectale, le principe actif, seul ou en association avec un autre principe actif, peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, intramusculaire, intraveineuse, intranasale ou intraoculaire et les formes d'administration rectale.

Dans les compositions pharmaceutiques de la présente invention, le principe actif ou les principes actifs sont généralement formulés en unités de dosage. L'unité de dosage contient de 0,5 à 1000 mg, avantageusement de 1 à 500 mg, de préférence de 2 à 200 mg d'antagoniste des récepteurs CB1 par unité de dosage pour les administrations quotidiennes.

Lorsqu'il s'agit de l'association de 2 principes actifs, l'unité de dosage contient de 0,5 à 600 mg, avantageusement de 1 à 400 mg, de préférence de 2 à 200 mg de composé antagoniste des récepteurs CB1 et de 0,5 à 600 mg, avantageusement de 2 à 400 mg préférentiellement de 10 à 250 mg de l'autre principe actif, notamment de $\beta_3$-agoniste.

Lorsque l'on prépare une composition solide sous forme de comprimés, on peut ajouter au(x) principe(s) actif(s) micronisé(s) ou non un agent mouillant tel que le laurylsulfate de sodium et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif ou les principes actifs avec un diluant tel qu'un glycol ou un ester de glycérol et en incorporant le mélange obtenu dans des gélules molles ou dures.

Une préparation sous forme de sirop ou d'élixir peut contenir le principe actif ou les principes actifs conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif ou les principes actifs en mélange avec des agents de dispersion ou des agents mouillants, ou des agents de mise en suspension, comme la polyvinylpyrrolidone ou polyvidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant, par exemple un alcool tel que l'éthanol ou un glycol tel que le polyéthylèneglycol ou le propylèneglycol, et un tensioactif hydrophile tel que le Tween® 80. Pour préparer une solution huileuse injectable par voie intramusculaire, on peut solubiliser le principe actif par un triglycéride ou un ester de glycérol.

Pour l'administration transdermique, on peut utiliser des patches sous forme multilaminée ou à réservoir dans lequel le principe actif est en solution alcoolique.

Le principe actif ou les principes actifs peuvent être formulés également sous forme de microcapsules ou microsphères, éventuellement avec un ou plusieurs supports ou additifs.

Le principe actif ou les principes actifs peuvent être également présentés sous forme de complexe avec une cyclodextrine, par exemple α-, β- ou γ- cyclodextrine, 2-hydroxypropyl-β-cyclodextrine ou méthyl-β-cyclodextrine.

Parmi les formes à libération prolongée utiles dans le cas de traitements chroniques, on peut utiliser des implants. Ceux-ci peuvent être préparés sous forme de suspension huileuse ou sous forme de suspension de microsphères dans un milieu isotonique.

Selon un autre aspect de l'invention, l'antagoniste des récepteurs CB1 et le régulateur des désordres métaboliques, notamment le $\beta_3$-agoniste, peuvent être administrés de manière simultanée, séquentielle ou étalée dans le temps pour le traitement des troubles de l'appétence, notamment pour le traitement des troubles des conduites alimentaires.

L'invention concerne donc également une trousse pour le traitement des troubles de l'appétence par administration simultanée, séquentielle ou étalée dans le temps d'un antagoniste des récepteurs CB1 et d'un régulateur des désordres métaboliques, notamment un $\beta_3$-agoniste, dans laquelle ledit antagoniste des récepteurs CB1 et ledit régulateur des désordres métaboliques, notamment ledit $\beta_3$-agoniste, sont dans des compartiments distincts et éventuellement dans des conditionnements différents.

Plus particulièrement, ladite trousse contient le N-pipéridino-5-(4-chlorophényl)-1-(2,4-dichlorophényl)-4-méthylpyrazole-3-carboxamide, un de ses sels pharmaceutiquement acceptables ou un de leurs solvates, et la N-[(2S)-7-éthoxycarbonylméthoxy-1,2,3,4-tétrahydronapht-2-yl]-(2R)-2-(3-chlorophényl)-2-hydroxyéthanamine ou un de ses sels pharmaceutiquement acceptables.

Selon un autre de ses aspects, l'invention concerne aussi une méthode pour le traitement des troubles de l'appétence, notamment pour le traitement des troubles des conduites alimentaires, qui consiste à administrer à un sujet en ayant besoin une quantité (thérapeutiquement) efficace d'un antagoniste des récepteurs $CB_1$ tel que défini précédemment. Ledit antagoniste des récepteurs $CB_1$ peut être avantageusement utilisé en association avec un régulateur des désordres métaboliques, notamment un $\beta_3$-agoniste, tel que défini précédemment. De manière particulière, l'antagoniste des récepteurs $CB_1$ et le régulateur des désordres métaboliques peuvent être administrés de manière simultanée, séquentielle ou étalée dans le temps.

ESSAI N° 1 : Effet du SR 141716 A sur la prise d'une solution de sucrose chez le rat.

L'expérience est réalisée selon W.C. Lynch et al., Physiol. Behav., 1993, 54, 877-880.

Des rats mâles Sprague-Dawley pesant 190 à 210 g sont dans un cycle lumineux normal (de 7 heures du matin à 19 heures) et reçoivent de l'eau et de la nourriture ad libitum.

Pendant 6 jours, entre 11 heures et 15 heures, la nourriture et les biberons d'eau sont enlevés et ils sont habitués à boire une solution de sucrose à 5 %.

Les rats buvant moins de 3 g de solution de sucrose sont éliminés.

Le septième jour on réalise l'essai en procédant de la façon suivante :

9 heures : retrait de la nourriture,
10 heures : administration du SR 141716 A par voie orale,
11 heures = T0: mise en place de biberons contenant une solution pesée de sucrose,

T0 + 1 heure, T0 + 2 heures, T0 + 3 heures, T0 + 4 heures :

mesure de la consommation de sucrose par pesée des biberons

TABLEAU 1

| Traitement p.o. | Nombre de rats | Consommation solution sucrose en g | | | |
|---|---|---|---|---|---|
| | | 1 Heure | 2 Heures | 3 Heures | 4 Heures |
| Véhicule 2 ml/kg | 8 | 11,33 ± 2,50 | 17,74 ± 4,00 | 22,50 ± 4,83 | 28,34 ± 5,01 |

(suite)

| Traitement p.o. | Nombre de rats | Consommation solution sucrose en g | | | |
|---|---|---|---|---|---|
| | | 1 Heure | 2 Heures | 3 Heures | 4 Heures |
| SR 141716 A 0,3 mg/kg | 6 | 5,18 ± 1,61 | 9,18 ± 2,12 | 12,49 ± 4,47 | 16,10 ± 3,95 |
| SR 141716 A 1 mg/kg | 6 | 3,27* ± 1,40 | 3,61** ± 1,40 | 5,65* ± 2,23 | 7,43** ± 2,81 |
| SR 141716 A 3 mg/kg | 6 | 2,95* ± 1,20 | 5,41* ± 1,33 | 6,96* ± 2,15 | 8,58** ± 2,92 |
| * p < 0,05 ; ** p < 0,01, test de Dunnett. | | | | | |

D'après les résultats reportés dans le TABLEAU 1, on constate que l'administration du SR 141716 A réduit très nettement la consommation d'eau sucrée, dès la dose de 0,3 mg/kg.

ESSAI N° 2 : Effet du SR 141716 A sur la consommation d'une solution alcoolique chez la souris.

Des souris mâles C 57 BL 6 (Iffa-Credo) sont isolées le jour de leur arrivée dans une animalerie en cycle inversé (nuit de 10 heures à 22 heures) avec 2 biberons remplis d'eau. Après 1 semaine, l'un des biberons d'eau est remplacé par un biberon rempli d'une solution d'alcool à 10 % pendant 6 heures de test. Chaque jour, 30 minutes avant la mise en contact avec le biberon d'alcool, les souris sont traitées par voie sous-cutanée avec du SR 141716 A. Les quantités d'alcool et d'eau bues sont mesurées au bout de 6 heures. Le test est répété pendant 4 jours.

TABLEAU 2

| Traitement mg/kg/sc SR 141716 A | Nombre de souris | Quantité d'alcool bue en g à J4 | Quantité d'eau bue en g |
|---|---|---|---|
| Véhicule | 20 | 1,9 ± 0,1 | 1,1 ± 0,1 |
| 0,1 | 10 | 1,4 ± 0,2 | 1,1 ± 0,3 |
| 0,3 | 10 | 1,3 ± 0,2 | 1,1 ± 0,3 |
| 1 | 10 | 1,1 ± 0,2** | 1,3 ± 0,1 |
| 3 | 10 | 1,0 ± 0,2** | 1,6 ± 0,3 |
| ** p < 0,01, test de Dunnett. | | | |

Les résultats montrent que pour les animaux traités, la consommation d'alcool diminue de façon très sensible : de 1,9 ± 0,1 g pour un animal non traité à 1,0 ± 0,2 g pour un animal ayant reçu 3 mg/kg de SR 141716 A ; parallèlement, la consommation d'eau a augmenté : de 1,1 ± 0,1 à 1,6 ± 0,3 g.

EXEMPLE 1 Gélule dosée à 1 mg d'antagoniste des récepteurs CB1.

| | |
|---|---|
| SR 141716 micronisé | 1,00 mg |
| Amidon de maïs | 51,00 mg |
| Lactose monohydrate | 103,33 mg |
| Polyvidone | 4,30 mg |
| Laurylsulfate de sodium | 0,17 mg |
| Carboxyméthyl cellulose de sodium réticulée | 8,50 mg |
| Eau purifiée : Q.S. pour granulation humide Stéarate de magnésium | 1,70 mg |

Pour une gélule blanc opaque n° 3 remplie à 170 mg

EXEMPLE 2 Gélule dosée à 10 mg d'antagoniste des récepteurs CB1.

| | |
|---|---|
| SR 141716 A micronisé | 10,00 mg |
| Amidon de maïs | 51,00 mg |

(suite)

| | |
|---|---|
| Lactose monohydrate | 94,33 mg |
| Polyvidone | 4,30 mg |
| Laurylsulfate de sodium | 0,17 mg |
| Carboxyméthylcellulose de sodium réticulée | 8,50 mg |
| Eau purifiée : Q.S. pour granulation humide Stéarate de magnésium | 1,70 mg |

Pour une gélule blanc opaque n° 3 remplie à 170 mg

EXEMPLE 3 Gélule dosée à 30 mg d'antagoniste des récepteurs CB1.

| | |
|---|---|
| SR 141716 micronisé | 30,00 mg |
| Amidon de maïs | 51,00 mg |
| Lactose monohydrate | 74,33 mg |
| Polyvidone | 4,30 mg |
| Laurylsulfate de sodium | 0,17 mg |
| Carboxyméthylcellulose de sodium réticulée | 8,50 mg |
| Eau purifiée : Q.S. pour granulation humide Stéarate de magnésium | 1,70 mg |

Pour une gélule blanc opaque n° 3 remplie à 170 mg

EXEMPLE 4 Comprimé dosé à 30 mg d'antagoniste des récepteurs CB1.

| | |
|---|---|
| SR 141716 micronisé | 30,00 mg |
| Lactose monohydrate | Q.S. |
| Amidon de maïs | 40,00 mg |
| Hydroxypropylméthylcellulose 6cP | 5,00 mg |
| Eau purifiée : Q.S. pour granulation humide Carboxyméthylcellulose de sodium réticulée | 10,00 mg |
| Stéarate de magnésium | 2,00 mg |

Pour un comprimé terminé à 200 mg.

EXEMPLE 5 Comprimé de 30 mg d'antagoniste des récepteurs CB1 et de 200 mg de $\beta_3$-agoniste.

| | |
|---|---|
| SR 141716 micronisé | 30,00 mg |
| SR 58611 A exprimé en base | 200,00 mg |
| Lactose monohydrate | Q.S. |
| Polyvidone | 15,00 mg |
| Eau purifiée : Q.S. pour granulation humide Carboxyméthylcellulose de sodium réticulée | 10,00 mg |
| Stéarate de magnésium | 5,00 mg |

Pour un comprimé terminé à 500 mg.

EXEMPLE 6 Comprimé de 10 mg d'antagoniste des récepteurs CB1 et de 100 mg de $\beta_3$-agoniste.

| | |
|---|---|
| SR 141716 micronisé | 10,00 mg |
| SR 58611 A exprimé en base | 100,00 mg |
| Amidon de maïs | 30 mg |
| Lactose monohydrate | Q.S. |
| Hydroxypropylméthylcellulose 6cP | 5,00 mg |
| Eau purifiée : Q.S. pour granulation humide Carboxyméthylamidon de sodium | 6,00 mg |
| Stéarate de magnésium | 3,00 mg |

Pour un comprimé terminé à 300 mg.

**Revendications**

1. Utilisation d'un antagoniste des récepteurs CB1 associé à un $\beta_3$-agoniste pour la préparation de médicaments utiles pour traiter les troubles de l'appétence.

2. Utilisation selon la revendication 1, pour la préparation de médicaments destinés à réguler les désirs de consommation.

3. Utilisation selon la revendication 1, pour la préparation de médicaments utiles dans le traitement des troubles liés à une substance.

4. Utilisation selon la revendication 1, pour la préparation de médicaments utiles dans le traitement des troubles des conduites alimentaires.

5. Utilisation selon la revendication 1, pour la préparation de médicaments utiles dans le traitement de l'obésité.

6. Utilisation selon la revendication 5, pour la préparation de médicaments utiles dans le traitement de l'obésité liée au diabète non insulino-dépendant.

7. Utilisation selon la revendication 1, pour la préparation de médicaments utiles dans le traitement de toute maladie se traduisant par un surpoids du malade.

8. Utilisation selon la revendication 1, pour la préparation de médicaments utiles dans le traitement de la boulimie.

9. Utilisation selon l'une quelconque des revendications 1 à 8, **caractérisée en ce que** l'antagoniste des récepteurs CB1 est un composé de formule:

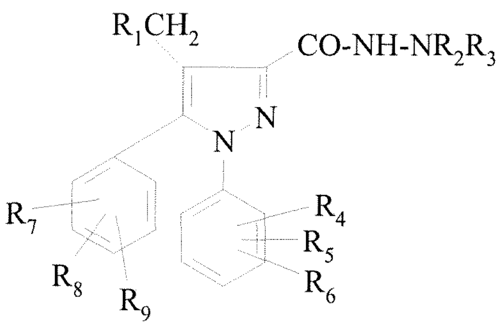

(II)

dans laquelle :

- $R_1$ représente l'hydrogène, un fluor, un hydroxy, un ($C_1$-$C_5$)alcoxy, un ($C_1$-$C_5$)alkylthio, un hydroxy($C_1$-$C_5$)alcoxy, un groupe -$NR_{10}R_{11}$, un cyano, un ($C_1$-$C_5$)alkylsulfonyle ou un ($C_1$-$C_5$)alkylsulfinyle ;

- $R_2$ et $R_3$ représentent un ($C_1$-$C_4$)alkyle ou ensemble avec l'atome d'azote auquel ils sont liés constituent un radical hétérocyclique, saturé ou insaturé, de 5 à 10 chaînons, non substitué ou substitué une ou plusieurs fois par un ($C_1$-$C_3$)alkyle ou par un ($C_1$-$C_3$)alcoxy ;
- $R_4$, $R_5$, $R_6$, $R_7$, $R_8$, $R_9$ représentent chacun indépendamment l'hydrogène, un halogène ou un trifluorométhyle, et lorsque $R_1$ représente un fluor, $R_4$, $R_5$, $R_6$, $R_7$, $R_8$ et/ou $R_9$ peuvent également représenter un fluorométhyle ; à la condition que l'un au moins des substituants $R_4$ ou $R_7$ soit différent de l'hydrogène ;
- $R_{10}$ et $R_{11}$ représentent chacun indépendamment l'hydrogène ou un ($C_1$-$C_5$)alkyle ou $R_{10}$ et $R_{11}$ ensemble avec l'atome d'azote auquel ils sont liés constituent un radical hétérocyclique choisi parmi pyrrolidin-1-yle, pipéridin-1-yle, morpholin-4-yle et pipérazin-1-yle, non substitué ou substitué par un ($C_1$-$C_4$)alkyle ;

un de ses sels ou un de leurs solvates.

**10.** Utilisation selon la revendication 9, **caractérisée en ce que** l'antagoniste des récepteurs CB1 est le N-pipéridino-5-(4-chlorophényl)-1-(2,4-dichlorophényl) -4-méthylpyrazole-3-carboxamide, un de ses sels pharmaceutiquement acceptables ou un de leurs solvates.

**11.** Utilisation selon la revendication 1, **caractérisée en ce que** ledit $\beta_3$-agoniste est un composé de formule :

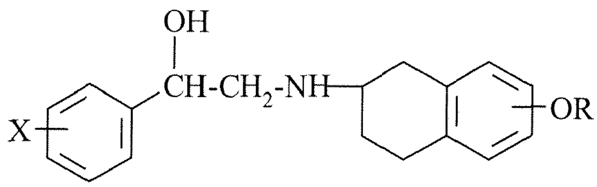
(III)

dans laquelle

- X représente l'hydrogène, un halogène, un trifluorométhyle ou un ($C_1$-$C_4$)alkyle;
- R représente l'hydrogène ou un méthyle, non substitué ou substitué par un carboxy ou un alcoxycarbonyle dont l'alcoxy est en ($C_1$-$C_6$) ;

ou un de ses sels pharmaceutiquement acceptables.

**12.** Utilisation selon la revendication 11, **caractérisée en ce que** ledit $\beta_3$-agoniste est la N-[(2S)-7-éthoxycarbonylméthoxy-1,2,3,4-tétrahydronapht-2-yl]-(2R)-2-(3-chlorophényl)-2-hydroxyéthanamine ou l'un de ses sels pharmaceutiquement acceptables.

**13.** Utilisation selon la revendication 1, **caractérisée en ce que** ledit $\beta_3$-agoniste est un composé de formule :

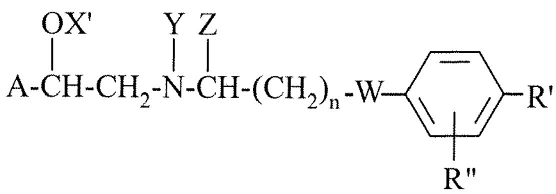
(IV)

dans laquelle

- n est 1, 2 ou 3;
- A représente un benzofuran-2-yle ou un phényle non substitué ou substitué par un ou deux atomes d'halogène ou par un ($C_1$-$C_4$)alkyle ou un trifluorométhyle;
- R' représente
  - l'hydrogène;
  - un ($C_1$-$C_6$)alkyle;
  - un groupe fonctionnel choisi parmi un groupe hydroxy, ($C_1$-$C_6$)alcoxy, ($C_2$-$C_6$)alcényloxy, ($C_2$-$C_6$)alcyny-

loxy, $(C_3-C_8)$cycloalkyloxy, $(C_3-C_8)$cycloalkyl$(C_1-C_6)$alcoxy, benzyloxy, phénoxy, mercapto, $(C_1-C_6)$alkylthio, $(C_2-C_6)$alcénylthio, $(C_2-C_6)$alcynylthio, $(C_3-C_8)$cycloalkylthio, $(C_3-C_8)$cycloalkyl$(C_1-C_6)$alkylthio, benzylthio, phénylthio, $(C_1-C_6)$alkylsulfinyle, $(C_2-C_6)$alcénylsulfinyle, $(C_2-C_6)$alcynylsulfinyle, $(C_3-C_8)$cycloalkylsulfinyle, $(C_3-C_8)$cycloalkyl$(C_1-C_6)$alkylsulfinyle, benzylsulfinyle, phénylsulfinyle, $(C_1-C_6)$alkylsulfonyle, $(C_2-C_6)$alcénylsulfonyle, $(C_2-C_6)$alcynylsulfonyle, $(C_3-C_8)$cycloalkylsulfonyle, $(C_3-C_8)$cycloalkyl$(C_1-C_6)$alkylsulfonyle, benzylsulfonyle, phénylsulfonyle, cyano, nitro, amino non substitué ou substitué par un ou deux radicaux, identiques ou différents, choisis parmi les groupes $(C_1-C_6)$alkyle, $(C_2-C_6)$alcényle, $(C_2-C_6)$alcynyle, $(C_3-C_8)$cycloalkyle, $(C_3-C_8)$cycloalkyl$(C_1-C_6)$alkyle, benzyle et phényle, carboxy, alcoxycarbonyle dont l'alcoxy est en $(C_1-C_6)$, $(C_2-C_6)$alcényloxycarbonyle, $(C_2-C_6)$alcynyloxycarbonyle, $(C_3-C_8)$cycloalkyloxycarbonyle, $(C_3-C_8)$cycloalkyl$(C_1-C_6)$alcoxycarbonyle, benzyloxycarbonyle, phénoxycarbonyle ou carbamoyle non substitué ou substitué sur le groupe amino par un ou deux radicaux, identiques ou différents, choisis parmi les groupes $(C_1-C_6)$alkyle, $(C_2-C_6)$alcényle, $(C_2-C_6)$alcynyle, $(C_3-C_8)$cycloalkyle, $(C_3-C_8)$cycloalkyl$(C_1-C_6)$alkyle, benzyle et phényle;

- un groupe R"' choisi parmi un groupe $(C_1-C_6)$alkyle substitué par un groupe fonctionnel, $(C_2-C_6)$alcényle substitué par un groupe fonctionnel, $(C_2-C_6)$alcynyle substitué par un groupe fonctionnel, phényl$(C_1-C_6)$ alkyle substitué sur le phényle par un $(C_1-C_6)$alkyle ou par un groupe fonctionnel, phényl$(C_2-C_6)$alcényle substitué sur le phényle par un $(C_1-C_6)$alkyle ou par un groupe fonctionnel, phényl$(C_2-C_6)$alcynyle substitué sur le phényle par un $(C_1-C_6)$alkyle ou par un groupe fonctionnel, benzyle substitué sur le phényle par un $(C_1-C_6)$alkyle ou par un groupe fonctionnel et phényle, non substitué ou substitué par un $(C_1-C_6)$alkyle ou par un groupe fonctionnel, le groupe fonctionnel étant tel que défini ci-dessus;
- un groupe O-R"', S-R"', SO-R"' ou $SO_2$-R"'dans lequel R"'est tel que défini ci-dessus;
- un groupe NR"'R°, où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- un groupe COOR"' ou un groupe CO-SR"' dans lequel R"'est tel que défini ci-dessus;
- un groupe CONR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- un groupe $SO_2$NR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- R" représente l'hydrogène; un halogène; un $(C_1-C_6)$alkyle; un groupe fonctionnel tel que défini ci-dessus; un groupe OR"', R"' étant tel que défini ci-dessus; un groupe COOR"', R"' étant tel que défini ci-dessus; ou un groupe CONR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- W représente une liaison directe ou un atome d'oxygène;
- X' représente l'hydrogène, un $(C_1-C_6)$alkyle ou un $(C_1-C_6)$alkylcarbonyle;
- Y représente l'hydrogène ou un groupe A'-CH(OH)-$CH_2$-, A' étant identique à A, mais autre que benzofuran-2-yle; ou bien
- X' et Y, pris ensemble, forment un groupe méthylène, éventuellement substitué par un alcoxycarbonyle dont l'alcoxy est en $(C_1-C_6)$; un groupe éthylène, éventuellement substitué par un groupe oxo; ou un groupe 1,3-propylène;
- Z représente l'hydrogène ou un $(C_1-C_6)$alkyle;

ou un de ses sels pharmaceutiquement acceptables.

**14.** Utilisation selon la revendication 1, **caractérisée en ce que** ledit $\beta_3$-agoniste est un composé de formule :

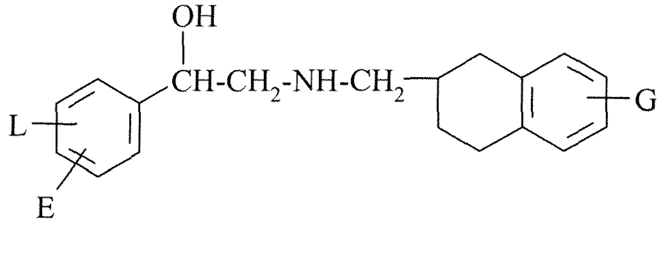

(V)

dans laquelle

- E représente l'hydrogène, un $(C_1-C_4)$alkyle, un $(C_1-C_4)$alkoxy, un phényle, un nitro, un atome d'halogène ou un trifluorométhyle,
- L représente l'hydrogène, un $(C_1-C_4)$alkyle, un $(C_1-C_4)$alkoxy, un phényle, un nitro ou un atome d'halogène ou bien, E et L , ensemble, représentent un groupe
- CH=CH-CH=CH- ou $-CH_2-CH_2-CH_2-CH_2-$, et
- G représente l'hydrogène, un atome de chlore, un hydroxy ou un groupe OG' où G' représente un $(C_1-C_4)$ alkyle non substitué ou substitué par un hydroxy, $(C_1-C_4)$alcoxy, $(C_1-C_4)$alcoxycarbonyle, carboxy ou $(C_3-C_7)$ cycloalkyle; un $(C_3-C_7)$cycloalkyl ou un $(C_2-C_4)$alcanoyle,

ou un de ses sels pharmaceutiquement acceptables.

**15.** Utilisation selon la revendication 1, **caractérisée en ce que** l'antagoniste des récepteurs CB1 est le N-pipéridino-5-(4-chlorophényl)-1-(2,4-dichlorophényl)-4-méthylpyrazole-3-carboxamide ou un de ses sels pharmaceutiquement acceptables ou un de leurs solvates et le $\beta_3$-agoniste est la N-[(2S)-7-éthoxycarbonylméthoxy-1,2,3,4-tétrahydronapht-2-yl)-(2R)-2-(3-chlorophényl)-2-hydroxyéthanamine ou un de ses sels pharmaceutiquement acceptables.

**16.** Composition pharmaceutique contenant un antagoniste des récepteurs CB1 et un $\beta_3$-agoniste avec un excipient pharmaceutique.

**17.** Composition pharmaceutique selon la revendication 16, **caractérisée en ce que** l'antagoniste des récepteurs $CB_1$ est un composé de formule :

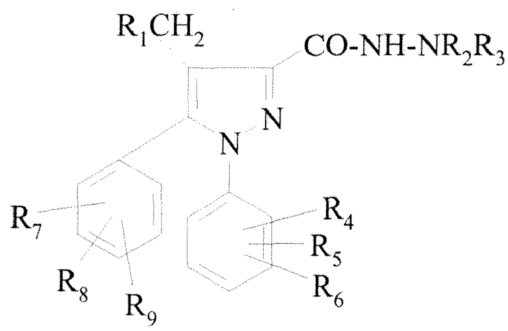

(II)

dans laquelle :

- $R_1$ représente l'hydrogène, un fluor, un hydroxy, un $(C_1-C_5)$alcoxy, un $(C_1-C_5)$alkylthio, un hydroxy$(C_1-C_5)$ alcoxy, un groupe $-NR_{10}R_{11}$, un cyano, un $(C_1-C_5)$alkylsulfonyle ou un $(C_1-C_5)$alkylsulfinyle ;
- $R_2$ et $R_3$ représentent un $(C_1-C_4)$alkyle ou ensemble avec l'atome d'azote auquel ils sont liés constituent un radical hétérocyclique, saturé ou insaturé, de 5 à 10 chaînons, non substitué ou substitué une ou plusieurs fois par un $(C_1-C_3)$alkyle ou par un $(C_1-C_3)$alcoxy ;
- $R_4$, $R_5$, $R_6$, $R_7$, $R_8$, $R_9$ représentent chacun indépendamment l'hydrogène, un halogène ou un trifluorométhyle, et lorsque $R_1$ représente un fluor, $R_4$, $R_5$, $R_6$, $R_7$, $R_8$ et/ou $R_9$ peuvent également représenter un fluorométhyle ; à la condition que l'un au moins des substituants $R_4$ ou $R_7$ soit différent de l'hydrogène ;
- $R_{10}$ et $R_{11}$ représentent chacun indépendamment l'hydrogène ou un $(C_1-C_5)$alkyle ou $R_{10}$ et $R_{11}$ ensemble avec l'atome d'azote auquel ils sont liés constituent un radical hétérocyclique choisi parmi pyrrolidin-1-yle, pipéridin-1-yle, morpholin-4-yle et pipérazin-1-yle, non substitué ou substitué par un $(C_1-C_4)$alkyle ;

un de ses sels ou un de leurs solvates.

**18.** Composition pharmaceutique selon la revendication 17, **caractérisée en ce que** l'antagoniste des récepteurs $CB_1$ est le N-pipéridino-5-(4-chlorophényl)-1-(2,4-dichlorophényl)-4-méthylpyrazole-3-carboxamide, un de ses sels pharmaceutiquement acceptables ou un de leurs solvates.

**19.** Composition pharmaceutique selon l'une quelconque des revendications 16 à 18, **caractérisée en ce que** le $\beta_3$-ago-

niste est un composé de formule :

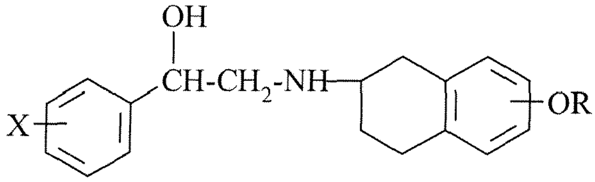

(III)

dans laquelle

- X représente l'hydrogène, un halogène, un trifluorométhyle ou un $(C_1\text{-}C_4)$alkyle;
- R représente l'hydrogène ou un méthyle, non substitué ou substitué par un carboxy ou un alcoxycarbonyle dont l'alcoxy est en $(C_1\text{-}C_6)$ ;

ou un de ses sels pharmaceutiquement acceptables.

**20.** Composition pharmaceutique selon l'une quelconque des revendications 16 à 18, **caractérisée en ce que** le $\beta_3$-agoniste est un composé de formule :

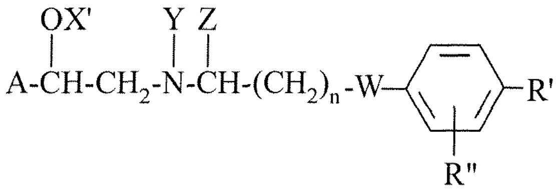

(IV)

dans laquelle

- n est 1, 2 ou 3;
- A représente un benzofuran-2-yle ou un phényle non substitué ou substitué par un ou deux atomes d'halogène ou par un $(C_1\text{-}C_4)$alkyle ou un trifluorométhyle;
- R' représente

  - l'hydrogène;
  - un $(C_1\text{-}C_6)$alkyle;
  - un groupe fonctionnel choisi parmi un groupe hydroxy, $(C_1\text{-}C_6)$alcoxy, $(C_2\text{-}C_6)$alcényloxy, $(C_2\text{-}C_6)$alcynyloxy, $(C_3\text{-}C_8)$cycloalkyloxy, $(C_3\text{-}C_8)$cycloalkyl$(C_1\text{-}C_6)$alcoxy, benzyloxy, phénoxy, mercapto, $(C_1\text{-}C_6)$alkylthio, $(C_2\text{-}C_6)$alcénylthio, $(C_2\text{-}C_6)$alcynylthio, $(C_3\text{-}C_8)$cycloalkylthio, $(C_3\text{-}C_8)$cycloalkyl$(C_1\text{-}C_6)$alkylthio, benzylthio, phénylthio, $(C_1\text{-}C_6)$alkylsulfinyle, $(C_2\text{-}C_6)$alcénylsulfinyle, $(C_2\text{-}C_6)$alcynylsulfinyle, $(C_3\text{-}C_8)$cycloalkylsulfinyle, $(C_3\text{-}C_8)$cycloalkyl$(C_1\text{-}C_6)$alkylsulfinyle, benzylsulfinyle, phénylsulfinyle, $(C_1\text{-}C_6)$alkylsulfonyle, $(C_2\text{-}C_6)$alcénylsulfonyle, $(C_2\text{-}C_6)$alcynylsulfonyle, $(C_3\text{-}C_8)$cycloalkylsulfonyle, $(C_3\text{-}C_8)$cycloalkyl$(C_1\text{-}C_6)$alkylsulfonyle, benzylsulfonyle, phénylsulfonyle, cyano, nitro, amino non substitué ou substitué par un ou deux radicaux, identiques ou différents, choisis parmi les groupes $(C_1\text{-}C_6)$alkyle, $(C_2\text{-}C_6)$alcényle, $(C_2\text{-}C_6)$alcynyle, $(C_3\text{-}C_8)$cycloalkyle, $(C_3\text{-}C_8)$cycloalkyl$(C_1\text{-}C_6)$alkyle, benzyle et phényle, carboxy, alcoxycarbonyle dont l'alcoxy est en $(C_1\text{-}C_6)$, $(C_2\text{-}C_6)$alcényloxycarbonyle, $(C_2\text{-}C_6)$alcynyloxycarbonyle, $(C_3\text{-}C_8)$cycloalkyloxycarbonyle, $(C_3\text{-}C_8)$cycloalkyl$(C_1\text{-}C_6)$alcoxycarbonyle, benzyloxycarbonyle, phénoxycarbonyle ou carbamoyle non substitué ou substitué sur le groupe amino par un ou deux radicaux, identiques ou différents, choisis parmi les groupes $(C_1\text{-}C_6)$alkyle, $(C_2\text{-}C_6)$alcényle, $(C_2\text{-}C_6)$alcynyle, $(C_3\text{-}C_8)$cycloalkyle, $(C_3\text{-}C_8)$cycloalkyl$(C_1\text{-}C_6)$alkyle, benzyle et phényle;
  - un groupe R''' choisi parmi un groupe $(C_1\text{-}C_6)$alkyle substitué par un groupe fonctionnel, $(C_2\text{-}C_6)$alcényle substitué par un groupe fonctionnel, $(C_2\text{-}C_6)$alcynyle substitué par un groupe fonctionnel, phényl$(C_1\text{-}C_6)$alkyle substitué sur le phényle par un $(C_1\text{-}C_6)$alkyle ou par un groupe fonctionnel, phényl$(C_2\text{-}C_6)$alcényle substitué sur le phényle par un $(C_1\text{-}C_6)$alkyle ou par un groupe fonctionnel, phényl$(C_2\text{-}C_6)$alcynyle substitué sur le phényle par un $(C_1\text{-}C_6)$alkyle ou par un groupe fonctionnel, benzyle substitué sur le phényle par un

($C_1$-$C_6$)alkyle ou par un groupe fonctionnel et phényle, non substitué ou substitué par un ($C_1$-$C_6$)alkyle ou par un groupe fonctionnel, le groupe fonctionnel étant tel que défini ci-dessus;
- un groupe O-R"', S-R"', SO-R"' ou $SO_2$-R"'dans lequel R"'est tel que défini ci-dessus;
- un groupe NR"'R°, où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- un groupe COOR"' ou un groupe CO-SR"' dans lequel R"'est tel que défini ci-dessus;
- un groupe CONR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- un groupe $SO_2$NR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;
- R" représente l'hydrogène; un halogène; un ($C_1$-$C_6$)alkyle; un groupe fonctionnel tel que défini ci-dessus; un groupe OR"', R"' étant tel que défini ci-dessus; un groupe COOR"', R"' étant tel que défini ci-dessus; ou un groupe CONR"'R° où R"' est tel que défini ci-dessus et R° représente l'hydrogène ou est tel que défini ci-dessus pour R"', ou bien R"' et R° forment, avec l'azote auquel ils sont liés, un groupe choisi parmi les groupes pyrrolidino, pipéridino et morpholino;

- W représente une liaison directe ou un atome d'oxygène;
- X' représente l'hydrogène, un ($C_1$-$C_6$)alkyle ou un ($C_1$-$C_6$)alkylcarbonyle;
- Y représente l'hydrogène ou un groupe A'-CH(OH)-$CH_2$-, A' étant identique à A, mais autre que benzofuran-2-yle; ou bien
- X' et Y, pris ensemble, forment un groupe méthylène, éventuellement substitué par un alcoxycarbonyle dont l'alcoxy est en ($C_1$-$C_6$); un groupe éthylène, éventuellement substitué par un groupe oxo; ou un groupe 1,3-propylène;
- Z représente l'hydrogène ou un ($C_1$-$C_6$)alkyle;

ou un de ses sels pharmaceutiquement acceptables.

**21.** Composition pharmaceutique selon l'une quelconque des revendications 16 à 18, **caractérisée en ce que** le $\beta_3$-agoniste est un composé de formule :

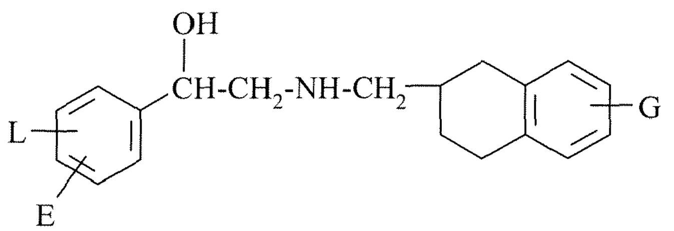

(V)

dans laquelle

- E représente l'hydrogène, un ($C_1$-$C_4$)alkyle, un ($C_1$-$C_4$)alkoxy, un phényle, un nitro, un atome d'halogène ou un trifluorométhyle,
- L représente l'hydrogène, un ($C_1$-$C_4$)alkyle, un ($C_1$-$C_4$)alkoxy, un phényle, un nitro ou un atome d'halogène ou bien, E et L , ensemble, représentent un groupe
- CH=CH-CH=CH- ou -$CH_2$-$CH_2$-$CH_2$-$CH_2$-, et
- G représente l'hydrogène, un atome de chlore, un hydroxy ou un groupe OG' où G' représente un ($C_1$-$C_4$) alkyle non substitué ou substitué par un hydroxy, ($C_1$-$C_4$)alcoxy, ($C_1$-$C_4$)alcoxycarbonyle, carboxy ou ($C_3$-$C_7$) cycloalkyle; un ($C_3$-$C_7$)cycloalkyl; un($C_2$-$C_4$)alcanoyle,

ou un de ses sels pharmaceutiquement acceptables.

**22.** Composition pharmaceutique selon la revendication 19, **caractérisée en ce que** le $\beta_3$-agoniste est la N-[(2S)-7-éthoxycarbonylméthoxy-1,2,3,4-tétrahydronapht-2-yl]-(2R)-2-(3-chlorophényl)-2-hydroxyéthanamine ou un de

ses sels pharmaceutiquement acceptables.

**23.** Composition pharmaceutique selon l'une quelconque des revendications 16 à 22, contenant de 0,5 à 600 mg d'antagoniste des récepteurs CB1 et de 0,5 à 600 mg de $\beta_3$-agoniste.

**24.** Composition pharmaceutique selon la revendication 23, contenant de 1 à 400 mg d'antagoniste des récepteurs CB1 et de 2 à 400 mg de $\beta_3$-agoniste.

**25.** Composition pharmaceutique selon la revendication 24, contenant de 2 à 200 mg d'antagoniste des récepteurs CB1 et de 10 à 250 mg de $\beta_3$-agoniste.

**26.** Trousse pour le traitement des troubles de l'appétence qui contient :

- un antagoniste des récepteurs $CB_1$, et
- un $\beta_3$-agoniste,

lesdits principes actifs étant dans des compartiments distincts et étant destinés à être administrés de manière simultanée, séquentielle ou étalée dans le temps.

**27.** Trousse selon la revendication 26, dans laquelle ledit antagoniste des récepteurs $CB_1$ est le N-pipéridino-5-(4-chlorophényl)-1-(2,4-dichlorophényl)-4-méthylpyrazole-3-carboxamide, un de ses sels pharmaceutiquement acceptables ou un de leurs solvates et ledit $\beta_3$ agoniste est la N-[(2S)-7-éthoxycarbonylméthoxy-1,2,3,4-tétrahydronapht-2-yl]-(2R)-2-(3-ehlorophényl)-2-hydroxyéthanamine ou un de ses sels pharmaceutiquement acceptables.

**28.** Trousse selon l'une des revendications 26 ou 27, dans laquelle lesdits principes actifs sont dans des conditionnements différents.

**29.** Utilisation selon la revendication 1 pour la préparation d'un médicament utile pour réguler le désir de consommation pour des éléments non essentiels de l'alimentation.

**30.** Utilisation selon la revendication 29 dans laquelle les éléments non essentiels de l'alimentation sont les sucres en excès, les carbohydrates en excès, l'alcool et les drogues.

**31.** Utilisation d'un antagoniste des récepteurs $CB_1$ pour la préparation d'un médicament utile pour supprimer l'appétence spontanée pour un ingrédient qui procure habituellement un plaisir.

**32.** Utilisation selon la revendication 31 dans laquelle l'ingrédient qui procure du plaisir est l'alcool ou le sucre.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 576357 A
- WO 9602248 A
- EP 656354 A
- EP 0211721 A
- EP 0303546 A
- EP 0255415 A
- EP 0436435 A
- EP 23385 A
- US 5061727 A
- EP 218440 A
- US 5321036 A
- EP 473285 A
- RO 402148
- WO 9635671 A
- WO 9635670 A
- WO 9616038 A
- WO 9604233 A
- WO 9533724 A
- WO 9529159 A
- EP 659737 A
- WO 9504047 A
- EP 516349 A
- EP 21636 A
- EP 7205 A
- JP 08198866 B
- JP 08165276 B
- JP 08157470 B
- WO 9616938 A
- EP 714883 A
- WO 9604234 A
- US 5488064 A
- US 5482971 A
- US 5491134 A
- ZA 9409874
- WO 9529903 A
- US 5461163 A
- WO 9525104 A
- JP 07112958 B
- WO 958527 A
- WO 9507284 A
- JP 07025756 B
- WO 9503289 A
- WO 9501170 A
- WO 9429290 A
- US 5373020 A
- JP 06293664 B
- WO 9412166 A
- US 5451677 A

### Littérature non-brevet citée dans la description


- **TUNER.** In Marijuana. IRL Press, 1985, vol. 84
- **DEVANE et al.** *Molecular Pharmacology,* 1988, vol. 34, 605-613
- **NYE et al.** *The Journal of Pharmacology and Experimental Therapeutics,* 1985, vol. 234, 784-791
- **KAMINSKI et al.** *Molecular Pharmacology,* 1992, vol. 42, 736-742
- **MUNRO et al.** *Nature,* 1993, vol. 365, 61-65
- *J. Pharmacol. Exp. Ther.,* 1988, vol. 247, 1046-1051
- *J. Pharmacol. Exp. Ther.,* 1993, vol. 264, 1352-1363
- **M. RINALDI-CARMONA et al.** *FEBS Lett.,* 1994, vol. 350, 240-244
- *J. Pain Symptom Manage,* 1995, vol. 10 (2), 89-97
- *J. Palliat. Care.,* 1994, vol. 10 (1), 14-18
- *Am. J. Clin. Nutr.,* 1992, vol. 55, 249S-251S
- **W.C. LYNCH et al.** *Physiol. Behav.,* 1993, vol. 54, 877-880